# EUROPEAN PATENT SPECIFICATION

(11) **EP 1 928 424 B1**
(45) Date of publication and mention of the grant of the patent: **24.08.2011**
(21) Application number: 06819063.6
(22) Date of filing: 29.09.2006
(51) Int. Cl.: A61K 9/16, A61K 31/137, A61K 9/48

(54) **CONTROLLED RELEASE PHARMACEUTICAL COMPOSITION OF VENLAFAXINE HYDROCHLORIDE, AND PROCESS FOR PREPARATION THEREOF**
PHARMAZEUTISCHE ZUSAMMENSETZUNG AUS VENLAFAXIN-HYDROCHLORID MIT KONTROLLIERTER FREISETZUNG UND HERSTELLUNGSVERFAHREN DAFÜR
LIBERATION PROGRESSIVE D'UNE PREPARATION PHARMACEUTIQUE HYDROCHLORURE DE VENLAFAXINE, ET SON PROCEDE DE PREPARATION

(30) Priority: 30.09.2005 IT FI20050206
(43) Date of publication of application: 11.06.2008
(73) Proprietor: Valpharma S.A., 47899 Serravalle (SM)
(72) Inventor: VALDUCCI, Roberto, I-47039 Savignano Sul Rubicone (IT); ALIGHIERI, Tiziano, I-47900 Rimini (IT); AVANESSIAN, Serozh, I-47827 Villa Verucchia (IT)
(74) Representative: Gervasi, Gemma
(86) International application number: PCT/EP2006/066906
(87) International publication number: WO 2007/039569

(56) References cited:
- WO-A-2004/047718
- US-A1- 2005 118 264
- MAKHIJA S N ET AL: "Once daily sustained release tablets of venlafaxine, a novel antidepressant" EUROPEAN JOURNAL OF PHARMACEUTICS AND BIOPHARMACEUTICS, ELSEVIER SCIENCE PUBLISHERS B.V., AMSTERDAM, NL, vol. 54, no. 1, July 2002 (2002-07), pages 9-15, XP004367686 ISSN: 0939-6411

## Description

### FIELD OF THE INVENTION

The present invention relates to the field of pharmaceutical compositions, and particularly to a new composition for controlled release of venlafaxine hydrochloride.

### STATE OF THE ART

Venlafaxine, that is 1-[2-dimethylamino-1-(4-methoxyphenyl)-ethyl]cyclohexanol, is a product with recognized activity in the treatment of depression disorders, but also in the treatment of other neurological diseases, such as epilepsy, Parkinson's disease, etc.

The recommended daily dose of the active principle ranges from 75 to 350 mg per day that should be administered in two or three divided doses. Such frequent administrations are particularly complicated, especially considering that patients are affected by neurological diseases. It is therefore evident the need to devise controlled release compositions of venlafaxine or its salts, that allow to avoid frequent drug administration and, at the same time, are suited for the particular pharmacokinetics of venlafaxine salts.

In fact, venlafaxine hydrochloride is a product with high solubility, therefore, if not appropriately formulated; it is released very rapidly, resulting in a high increase of plasma levels of the active principle within a short time after administration, followed by a drop below therapeutic levels approximately 12 hours after administration. Indeed, this makes multiple daily dosage necessary, which causes very undesirable side effects, such as nausea and vomiting in the majority of patients undergoing this dosing regimen.

For the reasons explained above, various controlled release formulations of venlafaxine hydrochloride have been devised to date, wherein the active principle is layered on an inert core and coated with one or more layers of polymeric materials which slow its release. In US 2005/118264 are descried pellets wherein Venlafaxine is applied to the inert core using suitable binders and then coated by an insulating layer consisting of polymers and waxes. WO 2004/047718 discloses compositions, comprising an inert core coated with Venlafaxine, blinder and anti lacking agent then coated with ethylcellulose and triacetin.

However, it should be said that such formulations are generally prepared by means of complicated and expensive processes, which are not suited for large scale preparation of uniform and stable pharmaceutical compositions.

### SUMMARY OF THE INVENTION

The Applicant has now found that it is possible to modify the release profile of venlafaxine hydrochloride, obtaining a controlled release of the active principle up to 24 hours after administration, by applying a lipophilic coating, as defined below, to the inert cores onto which the active compound has been previously layered. The active principle so formulated has shown *in vitro* an ideal type of release in order to achieve *in vivo* plasma levels suitable for a prolonged pharmaceutical effect, such that a single daily dose administration regimen can be instituted. Moreover, the present preparation process has shown a good lot-to-lot reproducibility, and a good stability of the compositions obtained.

Therefore, subject of the present invention is a pharmaceutical composition for controlled release of venlafaxine hydrochloride, comprising an inert core on which is applied the active principle venlafaxine hydrochloride, and a lipophilic coating comprising a lipophilic compound and a hardening agent.

The process for preparation of the above-mentioned pharmaceutical composition is a further subject of the invention.

Features and advantages of the invention will be illustrated in detail in the following description.

### SHORT DESCRIPTION OF THE FIGURES

Figure 1: shows the time profile of the plasma levels of venlafaxine.
Figure 2: shows the time profile of the plasma levels of O-desmethylvenlafaxine, the main metabolite of venlafaxine.

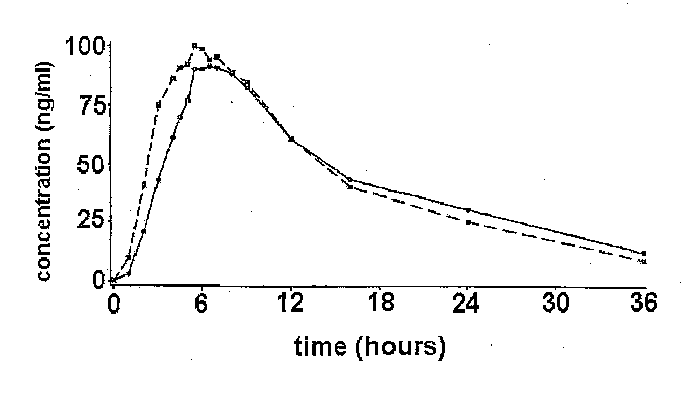

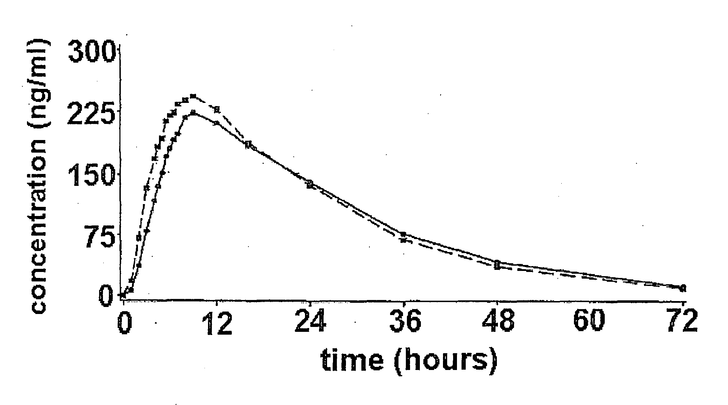

In both Figures 1 and 2 the symbol -■- indicates the curve obtained after administration of one capsule of the invention, prepared as described in Example 2, while the symbol -●- indicates the reference curve obtained after administration of a venlafaxine hydrochloride sustained release capsule known in the art; and t = 0 is the time of administration.

### DETAILED DESCRIPTION OF THE INVENTION

According to the invention, the weight ratio between the lipophilic compound and hardening agent can be comprised, for instance, between 1:1 and 1:20; preferably, the weight ratio between lipophilic compound and hardening agent is 1:10.

According to a preferred embodiment of the invention, the present composition comprises an amount of venlafaxine hydrochloride from 39.3% to 54.0% by weight in respect to the total weight of the composition, and from 1.35% to 2.10% by weight of lipophilic coating in respect to the total weight of the composition; the remaining part of the composition being constituted by the inert core.

Preferred hardening agents are cellulose derivatives, methacrylic acid and its copolymers, polyglycols, polyvinyls, and mixtures thereof.

In the present invention, by the expression "cellulose derivatives" are meant for example products selected from the group consisting of ethylcellulose, methylcellulose, hydroxypropylmethylcellulose, hydroxyethylcellulose, hydroxypropylcellulose, hydroxybutylcellulose, sodium carboxymethylcellulose, hydroxypropylmethylcellulose phtalate, and cellulose acetophtalate; while by the term "polyvinyls" are meant for instance, products chosen among polyvinylacetate, polyvinylpirrolidone and polyvinylacetophtalate. Polyethylenglycol is an example of polyglycols according to the invention.

Ethylcellulose is particularly preferred as hardening agent according to the present invention.

Lipophilic compounds of possible use according to the invention are chosen among paraffin, fatty acids with from 12 to 20 carbon atoms, and their mixtures; stearic acid is preferred.

Any inert pellet for pharmaceutical use can be used to prepare the core according to the invention; for instance, pellets made of saccharose and starch can be used. Preferably, the pellets of the invention have a particle size distribution comprised between 710 and 1340 µm.

Preferred pharmaceutical composition according to invention are those having the following composition expressed in percent by weight with respect to the total weight of the composition:

| Components | % by weight |
|---|---|
| venlafaxine hydrochloride | 49.10 |
| neutral pellets | 49.16 |
| ethylcellulose | 1.40 |
| stearic acid | 0.14 |
| talc | 0.20 |

The present preparation process includes the following steps:
i) application of the active principle on inert pellets by means of nebulization of an aqueous solution of venlafaxine hydrochloride;
ii) application of the lipophilic coating on the core comprising the active principle -coming from step i).

Preferably the application of the lipophilic coating of step ii) is carried out by means of Nebulization on the core coming from step i), of a solution comprising the lipophilic compound and the hardening agent in a suitable solvent. Preferably talc is added during said nebulization of she solution comprising the lipophilic compound and the hardening agent.

The application of the lipophilic compound in mixture with the hardening agent occurs in solution; the organic solvents used for solubilization are selected, for example, from the group consisting of acetone, ethanol, methylene chloride and their mixtures. Mixtures of ethanol and acetone are the preferred solvents for preparation of the solution of lipophilic compound to be applied on the active core. Said mixture of ethanol and acetone is preferably in a 1:1 weight ratio.

The pharmaceutical compositions according to the invention can comprise, in addition to the above mentioned active principle and components of the lipophilic coating, pharmaceutically acceptable excipients and/or diluents, chosen among those conventionally used for pharmaceutical compositions, in order to realize a composition suitable for controlled release oral administration.

The following examples are provided to illustrate the invention.

### EXAMPLE 1

### Preparation of active principle core

10 Kg Sugar Spheres neutral pellets (composition: 80% by weight saccharose, 20% starch), having particle size distribution between 710 and 1000 µm, are loaded in an automatic system GS model HP/M025.

On these neutral pellets a solution of venlafaxine HCl is applied, which is prepared by solubilization of 10 Kg of active principle in 10 Kg purified water.

The following Table 1 reports the operating conditions for application and the values of the main operating parameters set on the equipment.

**Table 1**

| Parameters | Set-up |
|---|---|
| Nozzle diameter | 1-0.8 mm |
| Nebulization pressure | 0.5-1.5 |
| Coater Speed | 16 rpm |
| Incoming air temperature | 60°C |
| Product temperature | 30-40°C |
| Nebulization cycle | continuous |

The so obtained pellets are sieved with a 1200 µm net and dried up for 12 hours at 60 °C.

### EXAMPLE 2

### Preparation of the controlled release coating

290 g of ethylcellulose and 29 g of stearic acid are added to 2.7 Kg of acetone and 2.7 Kg of 96% ethanol, and the mixture is placed under stirring until complete solubilization.

The so obtained solution has been used for application of a retardant film on the pellets prepared as described above in Example 1.

Talc is added in small amounts during nebulization of the solution, for a total amount of 40 g of talc added.

The following Table 2 reports the operating conditions for application of the retardant coat and the values of the main operating parameters set on the equipment.

**Table 2**

| Parameters | Set-up |
|---|---|
| Nozzle diameter | 1.0 mm |
| Nebulization pressure | 0.5-1.5 |
| Coater speed | 16 rpm |
| Incoming air temperature | 60-65°C |
| Product temperature | 30-45°C |
| Nebulization cycle | continuous |

The so obtained pellets are sieved with a 1340 µm net and dried up for 12 hours at 60°C. At the end of desiccation, pellets are encapsulated in "0" capsules, comprising 150 mg of venlafaxine corresponding to 169.7 mg of venlafaxine HCl.

### EXAMPLE 3

### Preparation of the active principle core

2 kg of Sugar Spheres neutral pellets, of the same type already described above in Example 1 are loaded in a Glatt fluid bed model GPCG 3, and a 50% venlafaxine HCl solution is applied on them.

At the end of the application of 4 Kg of solution, pellets are sieved with a 1200 µm net and dried up for 30 minutes at 60°C in the same fluid bed.

The following Table 3 reports the operating conditions for application and the values of the main operating parameters set on the equipment.

**Table 3**

| Parameters | Set-up |
|---|---|
| Nozzle diameter | 1.2 mm |
| Incoming air temperature | 35-50°C |
| Outgoing air temperature | 22-33°C |
| Product temperature | 21-34°C |
| Nebulization pressure | 0.5-1.5 |
| Pump speed | 4-9 rpm |

### EXAMPLE 4

### Preparation of the controlled release coating

58 g of ethylcellulose and 5.8 g of stearic acid are added to 0.54 Kg of acetone and 0.54 Kg of 96% ethanol, and the mixture is placed under stirring until complete solubilization.

The so obtained solution has been used for application of a retardant film on the pellets prepared as described above in Example 3 and placed again in fluid bed equipped with Wurster insert.

Talc is added in small amounts during nebulization of the solution, for a total amount of 8 g of talc added.

The following Table 4 reports the operating conditions for application of the retardant coat and the values of the main operating parameters set on the equipment.

**Table 4**

| Parameters | Set-up |
|---|---|
| Nozzle diameter | 1.2 mm |
| Incoming air temperature | 35-50°C |
| Outgoing air temperature | 22-33°C |
| Product temperature | 21-34°C |
| Nebulization pressure | 0.5-1.5 |

The so obtained pellets are sieved with a 1340 µm net and dried up for 12 hours at 60°C. At the end of desiccation, pellets are encapsulated in "0" capsules, containing 150 mg of venlafaxine corresponding to 169.7 mg of venlafaxine HCl.

### EXAMPLE 5

### Release test of the compositions of the invention

The capsules prepared as described above in Examples 2 and 4 have been analysed by the following method:
Apparatus: Basket 100 rpm; Solubilization medium: H₂O 900 ml
Results of the so performed tests are shown in the following Table 5.

**Table 5**

| Time (hours) | % release | |
|---|---|---|
| | Example 2: | Example 4 |
| 2 | 27 | 23 |
| 4 | 48 | 47 |
| 8 | 67 | 69 |
| 24 | 90 | 99 |

### EXAMPLE 6

### Bioequivalence test of the compositions of the invention

A bioequivalence study has been carried out on the capsules of the invention prepared as in Examples 2 and 4, having as reference the currently marketed sustained release venlafaxine hydrochloride 150 mg capsules.

The results of this study highlighted in Figures 1 and 2, show that the product of the invention is equivalent to the product that is currently marketed, both in terms of speed and extent of absorption of the active principle venlafaxine and in terms of speed and extent of formation of its main metabolite O-desmethylvenlafaxine.

## Claims

1. Controlled release pharmaceutical composition of venlafaxine hydrochloride, comprising:
- an inert core on which is applied a layer consisting of the active principle venlafaxine hydrochloride, and
- a lipophilic coating comprising a lipophilic compound and a hardening agent, wherein said lipophilic compound is selected in the group consisting of fatty acids with from 12 to 20 carbon atoms, and mixtures thereof.

2. Pharmaceutical composition according to claim 1, wherein the weight ratio between lipophilic compound and hardening agent in said lipophilic coating is comprised between 1:1 and 1:20.

3. Pharmaceutical composition according to claim 2, wherein said weight ratio between lipophilic compound and hardening agent is 1:10.

4. Pharmaceutical composition according to claim 1, wherein said hardening agent is selected from the group consisting of cellulose derivatives, methacrylic acid and its copolymers, polyglycols, polyvinyls, and mixtures thereof.

5. Pharmaceutical composition according to claim 4, wherein said hardening agent is selected from the group consisting of ethylcellulose, methylcellulose, hydroxypropylmethylcellulose, hydroxyethylcellulose, hydroxypropylcellulose, hydroxybutylcellulose, sodium carboxymethylcellulose, hydroxypropylmethylcellulose phtalate, cellulose acetophtalate, polyvinylacetate, polyvinylpirrolidone, polyvinylacetophtalate, polyethylenglycol and mixtures thereof.

6. Pharmaceutical composition according to claim 5, wherein said hardening agent is ethylcellulose.

7. Pharmaceutical composition according to claim 1, wherein said lipophilic compound is stearic acid.

8. Pharmaceutical composition according to claim 1, wherein said inert core essentially consists of neutral pellets composed of saccharose and starch, having a particle size distribution comprised between 710 and 1340 µm.

9. Pharmaceutical composition according to claims 1-8, further comprising pharmaceutically acceptable excipients and/or diluents.

10. Pharmaceutical composition according to claims 1-9, in the form of capsule.

11. Pharmaceutical composition according to claims 1-10, comprising an amount of venlafaxine hydrochloride from 39.3% to 54.0% by weight with respect to the total weight of the composition, and from 1.35% to 2.10% by weight of lipophilic coating with respect to the total weight of the composition; the remaining part of the composition being constituted by the inert core and possibly by pharmaceutically acceptable excipients and/or diluents.

12. Pharmaceutical composition according to claim 11, having the following composition expressed in percent by weight with respect to the total weight of the composition:
| Components | % by weight |
|---|---|
| venlafaxine hydrochloride | 49.10 |
| neutral pellets | 49.16 |
| ethylcellulose | 1.40 |
| stearic acid | 0.14 |
| talc | 0.20 |

13. Process for preparation of the pharmaceutical composition as defined in claims 1-12, comprising the following steps:
i) application of the active principle venlafaxine hydrochloride on the inert core by means of nebulization of an aqueous solution of venlafaxine hydrochloride;
ii) application on the core comprising the active principle, coming from step i), of the lipophilic coating comprising a lipophilic compound and a hardening agent.

14. Process according to claim 13, wherein application of the lipophilic coating in step ii) is carried out by means of nebulization on the core coming from step i), of a solution comprising the lipophilic compound and the hardening agent in a suitable solvent.

15. Process according to claim 14, wherein said solvent is an organic solvent selected from the group consisting of acetone, ethanol, methylene chloride and mixtures thereof.

16. Process according to claim 15, wherein said organic solvent is a mixture of ethanol and acetone in a 1:1 weight ratio.

17. Process according to claim 14, wherein talc is added during said nebulization of the solution comprising the lipophilic compound and the hardening agent.

## Patentansprüche

1. Pharmazeutische Zusammensetzung mit kontrollierter Freisetzung aus Venlafaxinhydrochlorid, welche enthält:
- einen inerten Kern, auf dem eine Schicht aufgebracht ist, welche aus dem aktiven Grundbestandteil Venlafaxinhydrochlorid besteht, und
- eine lipophile Beschichtung, welche eine lipophile Verbindung und einen Härter enthält, wobei die lipophile Verbindung aus der Gruppe ausgewählt ist, welche aus Fettsäuren mit 12 bis 20 Kohlenstoffatomen und Mischungen hiervon besteht.

2. Pharmazeutische Zusammensetzung nach Anspruch 1, wobei das Gewichtsverhältnis zwischen der lipophilen Verbindung und dem Härter in der lipophilen Beschichtung zwischen 1:1 1 und 1:20 beträgt.

3. Pharmazeutische Zusammensetzung nach Anspruch 2, wobei das Gewichtsverhältnis zwischen der lipophilen Verbindung und dem Härter 1:10 beträgt.

4. Pharmazeutische Zusammensetzung nach Anspruch 1, wobei das der Härter aus der Gruppe ausgewählt ist, welche aus Cellulosederivaten, Methacrylsäure und dessen Copolymeren, Polyglycolen, Polyvinylen und Mischungen hiervon besteht.

5. Pharmazeutische Zusammensetzung nach Anspruch 4, wobei der Härter aus der Gruppe ausgewählt ist, welche aus Ethylcellulose, Methylcellulose, Hydroxypropylmethylcellulose, Hydroxyethylcellulose, Hydroxypropylcellulose, Hydroxybutylcellulose, Natriumcarboxymethylcellulose, Hydroxypropylmethylcellulosephthalat, Celluloseacetophthalat, Polyvinylacetat, Polyvinylpyrrolidon, Polyvinylacetophthalat, Polyethylglycol und Mischungen hiervon besteht.

6. Pharmazeutische Zusammensetzung nach Anspruch 5, wobei der Härter Ethylcellulose ist.

7. Pharmazeutische Zusammensetzung nach Anspruch 1, wobei die lipophile Verbindung Stearinsäure ist.

8. Pharmazeutische Zusammensetzung nach Anspruch 1, wobei der inerte Kern im Wesentlichen aus neutralen Pellets, welche aus Saccharose und Stärke zusammengesetzt sind, mit einer Partikelgrößenverteilung zwischen 710 und 1340 µm besteht.

9. Pharmazeutische Zusammensetzung nach einem der Ansprüche 1 bis 8, welche des Weiteren pharmazeutisch akzeptable Hilfsstoffe und/oder Verdünnungsmittel enthält.

10. Pharmazeutische Zusammensetzung nach einem der Ansprüche 1 bis 9 in der Form einer Kapsel.

11. Pharmazeutische Zusammensetzung nach einem der Ansprüche 1 bis 10, welche Venlafaxinhydrochlorid in einer Menge zwischen 39,3 % und 54,0 Gew.-% bezogen auf das Gesamtgewicht der Zusammensetzung und zwischen 1,35 % und 2,10 Gew.-% der lipophilen Beschichtung bezogen auf das Gesamtgewicht der Zusammensetzung enthält, wobei der verbleibende Teil der Zusammensetzung aus einem inerten Kern und ggf. aus pharmazeutisch akzeptablen Hilfsstoffen und/oder Verdünnungsmitteln zusammengesetzt ist.

12. Pharmazeutische Zusammensetzung nach Anspruch 11 mit der nachfolgenden Zusammensetzung ausgedrückt in Gew.-% bezogen auf das Gesamtgewicht der Zusammensetzung:
| Bestandteile | Gewichts-% |
|---|---|
| Venlafaxinhydrochlorid | 49,10 |
| Neutrale Pellets | 49,16 |
| Ethylcellulose | 1,40 |
| Stearinsäure | 0,14 |
| Talk | 0,20 |

13. Verfahren zum Herstellen einer pharmazeutischen Zusammensetzung nach einem der Ansprüche 1 bis 12, welches die nachfolgenden Schritte umfasst:
i) Aufbringen des aktiven Grundbestandteils Venlafaxinhydrochlorid auf den inerten Kern durch Zerstäubung einer wässrigen Lösung von Venlafaxinhydrochlorid,
ii) Aufbringen der lipophilen Beschichtung, welche eine lipophile Verbindung und einen Härter enthält, auf den aus dem Schritt i) kommenden Kern, welcher den aktiven Grundbestandteil enthält.

14. Verfahren nach Anspruch 13, wobei das Aufbringen der lipophilen Beschichtung in dem Schritt ii) mittels Zerstäubung einer Lösung, welche die lipophile Verbindung und den Härter in einem geeigneten Lösungsmittel enthält, auf dem Kern, welcher aus dem Schritt i) kommt, durchgeführt wird.

15. Verfahren nach Anspruch 14, wobei das Lösungsmittel ein organisches Lösungsmittel ist, welches aus der Gruppe ausgewählt ist, welche aus Aceton, Ethanol, Methylenchlorid und Mischungen hiervon besteht.

16. Verfahren nach Anspruch 15, wobei das organische Lösungsmittel eine Mischung aus Ethanol und Aceton in einem Gewichtsverhältnis von 1:1 1 ist.

17. Verfahren nach Anspruch 14, wobei während der Zerstäubung der Lösung, welche die lipophile Verbindung und den Härter enthält, Talk zugegeben wird.

## Revendications

1. Libération contrôlée d'une composition pharmaceutique de chlorhydrate de venlafaxine, comprenant :
- un noyau inerte sur lequel est appliquée une couche consistant en le principe actif chlorhydrate de venlafaxine, et
- un revêtement lipophile comprenant un composé lipophile et un agent de durcissement, ledit composé lipophile étant choisi dans le groupe comprenant des acides gras ayant de 12 à 20 atomes de carbone, et des mélanges de ceux-ci.

2. Composition pharmaceutique selon la revendication 1, dans laquelle le rapport en poids entre le composé lipophile et l'agent de durcissement dans ledit revêtement lipophile est compris entre 1 : 1 et 1 : 20.

3. Composition pharmaceutique selon la revendication 2, dans laquelle ledit rapport en poids entre le composé lipophile et l'agent de durcissement est de 1 : 10.

4. Composition pharmaceutique selon la revendication 1, dans laquelle ledit agent de durcissement est choisi dans le groupe comprenant des dérivés de la cellulose, l'acide méthacrylique et ces copolymères, des polyglycols, des polyvinyles, et des mélanges de ceux-ci.

5. Composition pharmaceutique selon la revendication 4, dans laquelle ledit agent de durcissement est choisi dans le groupe comprenant l'éthylcellulose, la méthylcellulose, l'hydroxypropylméthylcellulose, l'hydroxyéthylcellulose, l'hydroxypropylcellulose, l'hydroxybutylcellulose, la carboxyméthylcellulose de sodium, le phtalate d'hydroxypropylméthylcellulose, l'acétophtalate de cellulose, l'acétate de polyvinyle, la polyvinylpyrrolidone, l'acétophtalate de polyvinyle, le polyéthylène glycol et des mélanges de ceux-ci.

6. Composition pharmaceutique selon la revendication 5, dans laquelle ledit agent de durcissement est de l'éthylcellulose.

7. Composition pharmaceutique selon la revendication 1, dans laquelle ledit composé lipophile est de l'acide stéarique.

8. Composition pharmaceutique selon la revendication 1, dans laquelle ledit noyau inerte consiste essentiellement en des pastilles neutres composées de saccharose et d'amidon, ayant une distribution de la taille des particules comprise entre 710 et 1 340 µm.

9. Composition pharmaceutique selon les revendications 1 à 8, comprenant en outre des excipients et/ou des diluants pharmaceutiquement acceptables.

10. Composition pharmaceutique selon les revendications 1 à 9, sous forme de capsule.

11. Composition pharmaceutique selon les revendications 1 à 10, comprenant une quantité de chlorhydrate de venlafaxine allant de 39,3 % à 54,0 % en poids par rapport au poids total de la composition, et de 1,35 % à 2,10 % en poids de revêtement lipophile par rapport au poids total de la composition ; la partie restante de la composition étant constituée du noyau inerte et éventuellement d'excipients et/ou de diluants pharmaceutiquement acceptables.

12. Composition pharmaceutique selon la revendication 11, ayant la composition suivante exprimée en pourcentage en poids par rapport au poids total de la composition :
| Composants | % en poids |
|---|---|
| chlorhydrate de venlafaxine | 49,10 |
| pastilles neutres | 49,16 |
| éthylcellulose | 1,40 |
| acide stéarique | 0,14 |
| talc | 0,20 |

13. Procédé de préparation de la composition pharmaceutique telle que définie dans les revendications 1 à 12, comprenant les étapes suivantes consistant à :
i) appliquer le principe actif chlorhydrate de venlafaxine sur le noyau inerte par le biais de la nébulisation d'une solution aqueuse de chlorhydrate de venlafaxine ;
ii) appliquer sur le noyau comprenant le principe actif, obtenu à l'étape i), le revêtement lipophile comprenant un composé lipophile et un agent de durcissement.

14. Procédé selon la revendication 13, dans lequel l'application du revêtement lipophile à l'étape ii) est réalisée par le biais de la nébulisation sur le noyau obtenu à l'étape i), d'une solution comprenant le composé lipophile et l'agent de durcissement dans un solvant approprié.

15. Procédé selon la revendication 14, dans lequel ledit solvant est un solvant organique choisi dans le groupe comprenant l'acétone, l'éthanol, le chlorure de méthylène et des mélanges de ceux-ci.

16. Procédé selon la revendication 15, dans lequel ledit solvant organique est un mélange d'éthanol et d'acétone dans un rapport en poids de 1 : 1.

17. Procédé selon la revendication 14, dans lequel est ajouté du talc pendant ladite nébulisation de la solution comprenant le composé lipophile et l'agent de durcissement.
